# EUROPÄISCHE PATENTSCHRIFT

(11) **EP 2 792 325 B1**
(45) Veröffentlichungstag und Bekanntmachung des Hinweises auf die Patenterteilung: **18.07.2018**
(21) Anmeldenummer: 14164608.3
(22) Anmeldetag: 14.04.2014
(51) Int. Cl.: A61B 17/70, A61B 90/00

(54) **Chirurgisches Instrument**
Surgical instrument
Instrument chirurgical

(30) Priorität: 20.04.2013 DE 102013207183
(43) Veröffentlichungstag der Anmeldung: 22.10.2014
(73) Patentinhaber: Silony Medical International AG, 8500 Frauenfeld (CH)
(72) Erfinder: Schäfer, Bernd, 73207 Plochingen (DE); Halm, Henry, 48151 Münster (DE)
(74) Vertreter: DREISS Patentanwälte PartG mbB

(56) Entgegenhaltungen:
- US-A1- 2003 225 408
- US-A1- 2006 079 909
- US-A1- 2012 253 413

## Beschreibung

Die Erfindung betrifft ein Instrument, für eine mit einem Wirbelsäulenstab zu verbindende Knochenschraube, wobei das Instrument ein im Wesentlichen zylindrisches und hohles Gehäuse sowie ein das Gehäuse axial durchsetzendes Einstellwerkzeug aufweist, welches an einem Abschnitt ein Außengewinde aufweist und in ein Innengewinde des Gehäuses eingeschraubt ist, und wobei das Gehäuse zangenartig aufgebaut ist und eine erste und eine zweite Backe aufweist, die gegenüber dem Benutzer jeweils ein proximales und ein distales Ende aufweisen und die distalen Enden am Kopf der Knochenschraube angreifen, wobei die beiden Backen über eine zwischen den distalen und proximalen Enden liegende, orthogonal zur Längsachse des Gehäuses verlaufende Schwenkachse gelenkig und einander nicht kreuzend miteinander verbunden sind, so dass ein Zusammendrücken der proximalen Enden der Backen ein Öffnen der distalen Enden der Backen bewirkt. Ein derartiges Instrument ist aus der EP 1 694 226 B1 oder der zur selben Familie gehörigen US 2006/0079909 A1 bekannt. Dieses Instrument dient dazu, eine in einem Knochen, insbesondere einem Wirbel verankerte Knochenschraube mit einem Wirbelsäulenstab zu verbinden. Die Knochenschraube weist hierfür einen Gabelkopf auf, in welchen der Wirbelsäulenstab eingelegt wird und dort mittels einer Madenschraube oder mittels einer Hutmutter fixiert wird. Um den Gabelkopf mit dem Wirbelsäulenstab zu fügen, benötigt man ein Instrument, mit welchem der Wirbelsäulenstab zwischen die beiden Schenkel des Gabelkopfes eingelegt oder eingeschoben wird. Das Instrument ist nach Art einer Zange aufgebaut und besitzt zwei Backen, deren proximale Enden dem Benutzer zugewandt sind und deren distale Enden am Kopf der Knochenschraube angreifen. Die beiden Backen sind über eine Querachse derart gelenkig oder verschwenkbar miteinander verbunden, dass ein Zusammendrücken der proximalen Enden ein Öffnen der distalen Enden bewirkt, so dass die Knochenschraube gegriffen werden kann.

Um den Wirbelsäulenstab in Richtung des Gabelkopfs der Knochenschraube zu bewegen, ist ein Einstellwerkzeug vorgesehen, welches zwischen die beiden Backen eingeschraubt wird. Hierfür weist das Einstellwerkzeug einen Abschnitt mit einem Außengewinde auf, der in ein Innengewinde, das an den Innenseiten der beiden Backen vorgesehen ist, eingeschraubt wird. Als nachteilig hat sich herausgestellt, dass das Innengewinde als zwei Gewindehälften ausgebildet ist, da es an den beiden Innenseiten der Backen vorgesehen ist. Einerseits ist die Herstellung des Innengewindes relativ aufwändig, da nach dem Zusammensetzen der beiden Backen die Gewindegänge, insbesondere deren Übergänge von der einen Backe zur anderen Backe exakt stimmen müssen, andererseits verursacht die gegenseitig schwenkbare Lagerung der beiden Backen ein relativ hohes Spiel zwischen dem Innengewinde der Backen und dem Außengewinde des Einstellwerkzeugs.
US 2008/0154277 A1 zeigt ein nicht gattungsgemäßes Instrument für entsprechende Zwecke, jedoch extrem komplexen Aufbaus, wobei eine Vielzahl von miteinander verschraubten Innen- und Außengewinden zwischen einer Vielzahl von tubusförmigen Abschnitten vorgesehen ist.
US 2003/0225408 A1 zeigt ein sehr komplexes nicht gattungsgemäßes Instrument ebenfalls zum Verbinden einer Knochenschraube mit einem Wirbelsäulenstab, bei dem ein Innengewinde zum Einschrauben eines das Gehäuse axial durchsetzenden Einstellwerkzeugs aber nicht bei den Backen sondern bei einem zusätzlichen hülsenförmigen Schaft ausgebildet ist.
US 2012/0253413 A1 offenbart ein Instrument mit sich kreuzenden Backen, wobei ein Innengewindeabschnitt an einem bezüglich einer Schwenkachse der Backen distalen Bereich einer Backe ausgebildet ist.
Der Erfindung liegt die Aufgabe zugrunde, ein Instrument der eingangs genannten Art derart weiterzubilden, dass das Einstellwerkzeug exakter im Gehäuse des Instruments geführt ist.
Diese Aufgabe wird erfindungsgemäß durch ein Instrument mit den Merkmalen des Anspruchs 1 gelöst.
Das Instrument besteht erfindungsgemäß aus einem Gehäuse, welches von einer ersten Backe und einer zweiten Backe gebildet wird, in dem das Innengewinde vorgesehen ist, in welches das Einstellwerkzeug mit seinem Außengewinde eingeschraubt wird. An diesem Gehäuse ist die andere Backe schwenkbar befestigt, so dass mit dieser Backe der Kopf der Knochenschraube gegriffen werden kann. Das Gehäuse ist in dem Bereich außerhalb des Abschnitts, welcher das Innengewinde aufweist, also ebenfalls als Backe ausgebildet und dient zusammen mit der anderen Backen als Greifwerkzeug.
Ein einfaches Lösen und Greifen des Kopfs der Knochenschraube wird dadurch erreicht, dass sich die beiden Backen nicht kreuzen. Hierdurch wird erreicht, dass dann, wenn die proximalen Enden der beiden Backen zusammengedrückt werden, die distalen Enden sich öffnen und der Kopf der Knochenschraube aufgenommen werden kann. Außerdem ist die Montage der erfindungsgemäßen Backen wesentlich einfacher, als bei sich kreuzenden Backen.
Bei einer Weiterbildung ist vorgesehen, dass bezüglich der Längsachse des Innengewindes eine Backe starr und die andere Backe schwenkbar ist. Die starre Backe bildet somit das Gehäuse oder ist Teil des Gehäuses des Instruments und trägt das Innengewinde für das Einstellwerkzeug. Erfindungsgemäß überragt die das Innengewinde aufweisende Backe die andere Backe in axialer Richtung, mit ihrem Innengewinde, also mit einem das Innengewinde umfassenden hülsenförmigen Abschnitt. Die andere Backe ist in einen Ausschnitt der starren Backe eingefügt, und vervollständigt dadurch den Mantel der starren Backe. Dieser Ausschnitt ist gewindefrei.
Es erweist sich weiter als vorteilhaft, dass die eine das Innengewinde des Gehäuses bildende Backe als ein einziges einstückiges Teil ausgebildet ist. Es ist hierbei mit einem halbschalenförmigen Bereich und einem hülsenförmigen Bereich, der das Innengewinde trägt, ausgebildet und kann auf besonders wirtschaftliche Weise hergestellt werden. Die beiden Backen sind aneinander und zueinander schwenkbar angelenkt und bilden das Gehäuse. Weitere Fügeverbindungen zur Bildung des Gehäuses sind nicht erforderlich.

Bei einem bevorzugten Ausführungsbeispiel der Erfindung ist vorgesehen, dass das Einstellwerkzeug in Achsrichtung zumindest zweiteilig aufgebaut ist und einen, mit dem Außengewinde versehenen drehbaren Abschnitt und einen am Wirbelsäulenstab angreifenden drehfesten Abschnitt aufweist. Auf diese Weise wird die Drehbewegung des Einstellwerkzeugs, mit welcher dieses in das Gehäuse des Instruments eingeschraubt wird, nicht auf den Wirbelsäulenstab übertragen, sondern lediglich die axiale Verlagerung des Einstellwerkzeugs innerhalb des Gehäuses des Instruments. Der Wirbelsäulenstab wird somit schonend in den Gabelkopf gedrängt. Dabei sind die beiden Abschnitte des Einstellwerkzeugs zwar gegeneinander verdrehbar, aber nicht voneinander lösbar. Das Einstellwerkzeug ist also nach wie vor einteilig.

Der drehfeste Abschnitt wird gemäß einer bevorzugten Ausführungsform in Längsrichtung zwischen den beiden Backen geführt. Hierfür weist er z.B. zwei radial nach außen abragende Zapfen auf, die zwischen die beiden Backen eingreifen und eine Verdrehung des drehfesten Abschnittes gegenüber dem Gehäuse verhindern.

Bei einer vorteilhaften Variante greift beim Zusammendrücken der proximalen Enden der Backen die eine Backe in die Gewindeflucht des Außengewindes des Einstellwerkzeugs ein. Ist das Einstellwerkzeug noch nicht so weit in das Gehäuse eingeschraubt, dass dessen Außengewinde über das oder aus dem Innengewinde vorsteht, dann kann die bewegliche Backe noch eingedrückt werden. Es kann also mit dem Instrument die Knochenschraube an deren Kopf gegriffen werden. Wird nunmehr das Einstellwerkzeug weiter in das Innengewinde der einen Backe eingeschraubt und tritt das Außengewinde über das Innengewinde in distaler Richtung hervor. Dann blockiert das Einstellwerkzeug ein Zusammendrücken der beiden Backen, insbesondere kann das proximale Ende der beweglichen Backe nicht mehr eingedrückt werden, da es am Innengewinde des Einstellwerkzeugs anschlägt. Dies bewirkt, dass die beiden Backen nicht mehr zusammengedrückt werden können, so dass die Knochenschraube sicher vom Instrument gehalten wird. Ein versehentliches Lösen der Knochenschraube vom Instrument wird dadurch verhindert.

Es kann vorgesehen sein, dass das Einstellwerkzeug hohl ausgebildet ist und der Hohlkörper einen Außendurchmesser aufweist, der im Wesentlichen dem Außendurchmesser des Kopfes der Knochenschraube entspricht. Das Einstellwerkzeug besitzt eine Wanddicke, die im Wesentlichen der Wanddicke der beiden Schenkel des Gabelkopfes entspricht, so dass durch das Einstellwerkzeug hindurch eine Madenschraube zwischen die beiden Schenkel in den Gabelkopf der Knochenschraube eingeschraubt und dadurch der Wirbelsäulenstab im Kopf der Knochenschraube fixiert werden kann.

Bei einer Ausführungsform weist das Außengewinde des Einstellwerkzeugs einen Durchmesser auf, der größer ist, als der Innendurchmesser oder der Abstand der zusammengedrückten proximalen Enden der Backen. Hierdurch wird, wie bereits erwähnt, verhindert, dass bei in das Gehäuse eingeschraubtem Einstellwerkzeug die beiden proximalen Enden der Backen versehentlich zusammengedrückt werden. Ein versehentliches Lösen der Knochenschraube vom Instrument ist somit nicht möglich.

Schließlich weist das Gehäuse und/oder das Einstellwerkzeug Aufnahmen für zusätzliche Halte- und/oder Drehgriffe auf. Mit diesen Halte- und Drehgriffen kann der Hebel einerseits zum Halten des Instruments, andererseits zum Betätigen des Instruments vergrößert und dadurch das Instrument feinfühliger betätigt werden.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel im Einzelnen beschrieben ist. Dabei können die in der Zeichnung dargestellten sowie in den Ansprüchen und in der Beschreibung erwähnten Merkmale jeweils einzeln für sich oder in beliebiger Kombination erfindungswesentlich sein.

In der Zeichnung zeigen:
- Figur 1: eine perspektivische Ansicht des Instruments in Gebrauchslage;
- Figur 2: eine Seitenansicht des Instruments in Richtung des Pfeils II gemäß Figur 1;
- Figur 3: eine Draufsicht auf das Instrument in Richtung des Pfeils III gemäß Figur 1;
- Figur 4: eine Explosionsdarstellung des Instruments;
- Figur 5: eine perspektivische Ansicht des Instruments mit vollständig eingeschraubtem Einstellwerkzeug; und
- Figur 6: eine perspektivische Darstellung des Instruments mit zusätzlichen Halte- und Drehgriffen.

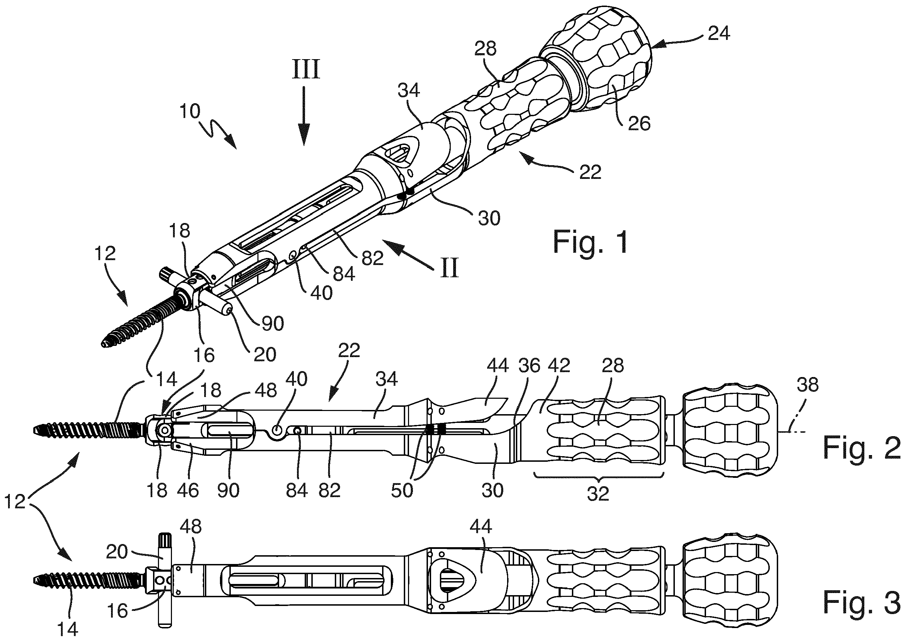

Die Figur 1 zeigt ein insgesamt mit 10 bezeichnetes Instrument, mit welchem eine Knochenschraube 12 gegriffen werden kann. Diese Knochenschraube 12 ist im dargestellten Ausführungsbeispiel als Polyaxialschraube mit einem Schraubenschaft 14 und einem Gabelkopf 16 ausgebildet. Dieser Gabelkopf 16 weist zwei parallele Schenkel 18 auf, die einen Wirbelsäulenstab 20 umgreifen. Das Instrument 10 besitzt ein Gehäuse 22, welches von einem Einstellwerkzeug 24 axial durchgriffen wird. Am Einstellwerkzeug 24 befindet sich ein integrierter Drehgriff 26, mit welchem das Einstellwerkzeug 24 im Gehäuse 22 gedreht werden kann. Das Gehäuse 22 wird seinerseits an einem integrierten Haltegriff 28 festgehalten.

Wie aus Figur 2 ersichtlich, ist das Gehäuse 22 im Wesentlichen zweiteilig aufgebaut und besitzt eine erste Backe 30, die einen Abschnitt 32 aufweist, an welchem der Haltegriff 28 ausgebildet ist. Vervollständigt wird das Gehäuse 22 von einer zweiten Backe 34, die in einen Ausschnitt 36 der ersten Backe 30 eingepasst ist. Die beiden Backen 30 und 34 sind über ein quer zur Längsachse 38 verlaufendes Schwenklager 40 derart gelenkig miteinander verbunden, dass beim Zusammendrücken der beiden proximalen Enden 42 und 44, wobei insbesondere das proximale Ende 44 in den Ausschnitt 36 eingedrückt wird, die distalen Enden 46 und 48 sich voneinander entfernen, so dass der Gabelkopf 16 zwischen die beiden distalen Enden 46 und 48 geschoben werden kann. Im Bereich der proximalen Enden 42 und 44 befinden sich Rückstellfedern 50, die die beiden proximalen Enden 42 und 44 in ihre Ausgangslage zurückbringen. Wie aus den Figuren 1 bis 3 leicht erkennbar, wird das Instrument 10 mit einer Hand betätigt, indem der Handgriff 28 mit vier Fingern und dem Handballen umgriffen und das proximale Ende 44 mit dem Daumen dieser Hand betätigt, insbesondere eingedrückt wird. Auf diese Weise wird der Gabelkopf 16 der Knochenschraube 12 gegriffen.

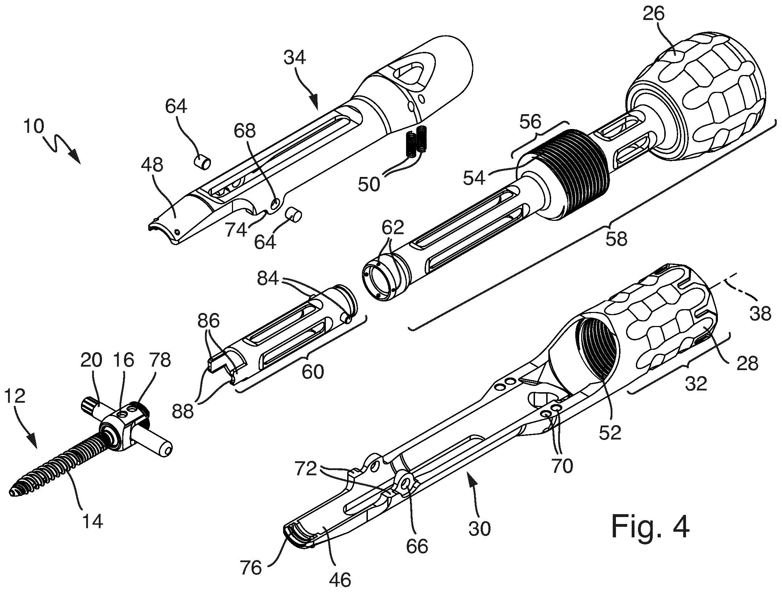

Die Figur 4 zeigt das Instrument 10 in seinen Einzelteilen und es ist ein Innengewinde 52 im Abschnitt 32 der ersten Backe 30, d.h. im Haltegriff 28, erkennbar. In dieses Innengewinde 52 wird ein Außengewinde 54 eingeschraubt, welches an einem Abschnitt 56 des Einstellwerkzeugs 24 vorgesehen ist.

Ferner ist erkennbar, dass das Einstellwerkzeug 24 zweiteilig aufgebaut ist und einen, das Außengewinde 54 aufweisenden ersten Abschnitt 58 und einen zweiten Abschnitt 60 aufweist, die derart miteinander verbunden sind, dass sie gegeneinander verdreht werden können, aber in axialer Richtung unlösbar sind. Dies erfolgt durch eine Verstemmung, von welcher Verstemmpunkte 62 erkennbar sind. Das Schwenklager 40 wird von zwei Lagerbolzen 64 gebildet, die in entsprechende Aufnahmen 66 und 68 in den beiden Backen 30 und 34 eingreifen. Die Rückstellfedern 50 sind in Einsenkungen 70 gelagert. Eine Endlage der beiden Backen 30 und 34 wird über Anschläge 72 und 74 erreicht, die ein vollständiges Zuschwenken der distalen Enden 46 und 48 verhindern. Diese Enden 46 und 48 besitzen an der Innenfläche Umfangsrillen 76 zum Greifen einer Hinterschneidung 78 am Gabelkopf 16 der Knochenschraube 12.

Sind die beiden Backen 30 und 34, wie in den Figuren 1 und 2 dargestellt, gefügt, bildet sich zwischen diesen beiden Backen 30 und 34 ein Längsschlitz 82, in welchen Zapfen 84, die vom zweiten Abschnitt 60 radial abragen, eingreifen.

Diese Zapfen 84 verhindern eine Verdrehung dieses zweiten Abschnitts 60, so dass dieser lediglich in Richtung der Längsachse 38 bewegt werden kann. Der erste Abschnitt 58 ist dagegen verschraubbar in der ersten Backe 30 geführt.

Am distalen Ende des zweiten Abschnitts 60 befinden sich zwei Fortsätze 86, die an ihrem freien Ende jeweils eine Einsenkung 88 aufweisen, mit welchen der Wirbelsäulenstab 20 in Richtung des Gabelkopfs 16 gedrückt werden kann. Dieser Wirbelsäulenstab 20 ist innerhalb eines Längsschlitzes 90 (siehe Figuren 1 und 2), der ebenfalls zwischen den beiden Backen 30 und 34 gebildet wird, verlagerbar und wird beim Einschrauben des Einstellwerkzeugs 24 in Richtung der Knochenschraube 12 bewegt.

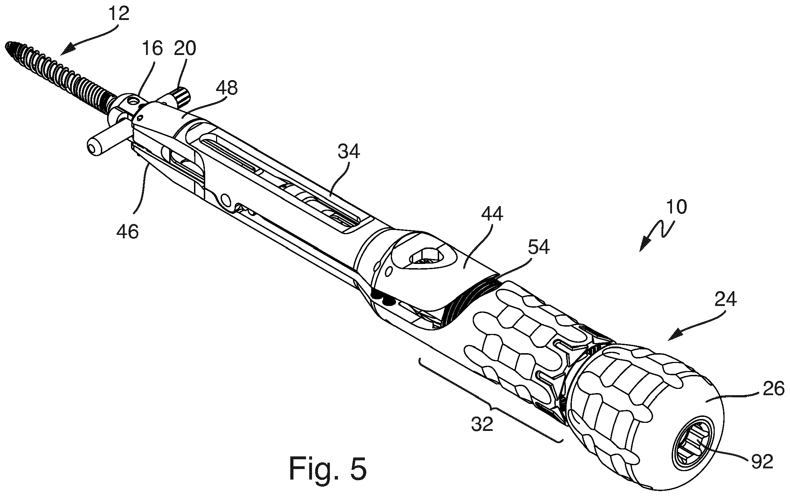

In der Figur 5 ist das Einstellwerkzeug 24 weiter eingeschraubt, als in der Figur 1. Es ist erkennbar, dass das Außengewinde 54 aus dem Abschnitt 32, in welchem das Innengewinde 52 vorgesehen ist, axial hervortritt, so dass das Außengewinde 54 in den Schwenkbereich des proximalen Endes 44 der zweiten Backe 34 gelangt. Diese Backe 34 kann nun nicht mehr betätigt, insbesondere das proximale Ende 44 nicht mehr eingedrückt werden, so dass keine Gefahr besteht, dass der Gabelkopf 16 der Knochenschraube 12 von den distalen Enden 46 und 48, d.h. vom Instrument 10, frei kommt. Bei eingeschraubtem Einstellwerkzeug 24 ist also die Knochenschraube 12 am Instrument 10 gesichert.

Es kann nunmehr über die zentrale Öffnung 92 im Handgriff 28 mittels eines geeigneten Werkzeugs eine Madenschraube in Richtung der Längsachse 38 eingeführt und in den Gabelkopf 16 eingeschraubt und dadurch der Wirbelsäulenstab 20 an der Knochenschraube 12 fixiert werden.

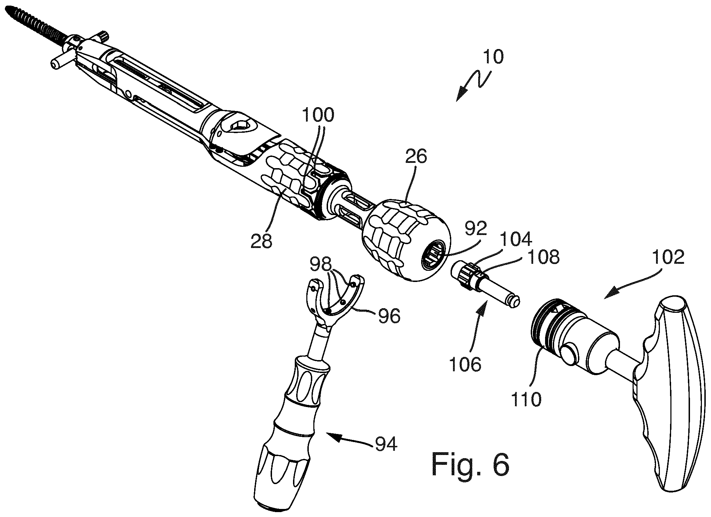

Sollten höhere Kräfte oder exakte Drehmomente aufgebracht werden müssen, kann das Instrument 10, wie in Figur 6 dargestellt, mit zusätzlichen externen Werkzeugen bestückt werden. Einerseits kann ein zusätzlicher, externer Handgriff 94 am Haltegriff 28 befestigt werden, indem eine Aufnahmegabel 96 mit radial nach innen vorspringenden Zapfen 98 derart axial auf den Handgriff 28 aufgeschoben wird, dass die Zapfen 98 in über den Umfang verteilt angeordnete, in Richtung des proximalen Endes 42 axial offene Zapfenaufnahmen 100 eingreifen.

Außerdem kann der Drehgriff 26 mit einem externen Drehgriff 102 bestückt werden, indem in die zentrale Öffnung 92 ein eine Verzahnung 104 aufweisender Adapter 106 eingesetzt wird, auf welchen der Drehgriff 102 aufgeschoben wird, der an Flanken 108 formschlüssig mit dem Adapter 106 verbunden werden kann. Der Drehgriff 102 besitzt einen Drehmomentbegrenzer 110 und kann von Linksdrehung auf Rechtsdrehung umgeschaltet werden.

Mit dem erfindungsgemäßen Instrument 10 kann eine Knochenschraube 12 auf einfache Weise mit einem Wirbelsäulenstab 20 verbunden werden.

## Patentansprüche

1. Instrument (10) zum Verbinden einer Knochenschraube (12)mit einem Wirbelsäulenstab (20), wobei das Instrument (10) ein im Wesentlichen zylindrisches und hohles Gehäuse (22) sowie ein das Gehäuse (22) axial durchsetzendes Einstellwerkzeug (24) aufweist, welches an einem Abschnitt (56) ein Außengewinde (54) aufweist und in ein Innengewinde (52) des Gehäuses (22) eingeschraubt ist, wobei das Innengewinde (52) eine Längsachse (38) des Gehäuses (22) definiert, und wobei das Gehäuse (22) zangenartig aufgebaut ist und eine erste und eine zweite Backe (30, 34) aufweist, die das Gehäuse (22) bilden und die gegenüber dem Benutzer jeweils ein proximales und ein distales Ende (42, 44, 46, 48) aufweisen, wobei die proximalen Enden (42, 44) der Backen zum Ergreifen des Instruments durch den Benutzer dienen und wobei die distalen Enden (46, 48) der Backen (30, 34) am Kopf (16) der Knochenschraube (12) angreifen, wobei die beiden Backen (30, 34) über eine zwischen den distalen und proximalen Enden (42, 44, 46, 48) liegende, orthogonal zur Längsachse (38) des Gehäuses (22) verlaufende Schwenkachse (40) gelenkig und einander nicht kreuzend miteinander verbunden sind, so dass ein Zusammendrücken der proximalen Enden (42, 44) der Backen (30, 34) ein Öffnen der distalen Enden (46, 48) der Backen (30, 34) bewirkt, **dadurch gekennzeichnet, dass** das Innengewinde (52) des Gehäuses (22) an dem proximalen Ende (42) einer einzigen Backe (30) ausgebildet ist, indem das proximale Ende (42) dieser Backe (30) das proximale Ende (44) der anderen Backe (34) in Richtung der Längsachse (38) mit einem das Innengewinde (52) tragenden Abschnitt (32) überragt.

2. Instrument nach einem der vorgehenden Ansprüche, **dadurch gekennzeichnet, dass** bezüglich der Längsachse (38) des Innengewindes (52) eine Backe (30) starr und eine Backe (34) schwenkbar ist.

3. Instrument nach einem der vorgehenden Ansprüche, **dadurch gekennzeichnet, dass** die eine Backe (30), an der das Innengewinde (52) des Gehäuses (22) ausgebildet ist, als ein einziges einstückiges Teil ausgebildet ist und zusammen mit der anderen Backe (34) das Gehäuse (22) bildet.

4. Instrument nach einem der vorhergehenden Ansprüche, **dadurch gekennzeichnet, dass** das Einstellwerkzeug (24) in Richtung der Längsachse (38) zumindest zweiteilig aufgebaut ist und einen mit dem Außengewinde (54) versehenen drehbaren Abschnitt (58) und einen am Wirbelsäulenstab angreifenden und am Gehäuse (22) drehfest gehaltenen Abschnitt (60) aufweist.

5. Instrument nach Anspruch 5, **dadurch gekennzeichnet, dass** der am Gehäuse (22) drehfest gehaltene Abschnitt (60) in Richtung der Längsachse (38) und zwischen den beiden Backen (30, 34) geführt ist.

6. Instrument nach einem der vorhergehenden Ansprüche, **dadurch gekennzeichnet, dass** beim Zusammendrücken der proximalen Enden (42, 44) der Backen (30, 34) die Backe (34) in die Gewindeflucht des Außengewindes (54) des Einstellwerkzeugs (24) eingreift.

7. Instrument nach einem der vorhergehenden Ansprüche, **dadurch gekennzeichnet, dass** das Außengewinde (54) des Einstellwerkzeugs (24) einen Durchmesser aufweist, der größer ist als der Abstand der zusammengedrückten proximalen Enden (42, 44) der Backen (30, 34).

8. Instrument nach einem der vorhergehenden Ansprüche, **dadurch gekennzeichnet, dass** das Gehäuse (22) und/oder das Einstellwerkzeug (24) Aufnahmen (92, 100) für zusätzliche Halte- (94) und/oder Drehgriffe (102) aufweist.

## Claims

1. Instrument (10) for connecting a bone screw (12) to a spinal column rod (20), wherein the instrument (10) comprises a substantially cylindrical and hollow housing (22) and an adjustment tool (24) which passes axially through the housing (22) and which has an external thread (54) on one portion (56) and is screwed into an internal thread (52) of the housing (22), wherein the internal thread (52) defines a longitudinal axis (38) of the housing (22), and wherein the housing (22) is formed as a pincer having a first and a second jaw (30, 34) which form the housing (22) and each have a proximal and a distal end (42, 44, 46, 48) relative to the user, wherein the proximal ends (42, 44) of the jaws serve for gripping the instrument by the user, and wherein the distal ends (46, 48) of the jaws (30, 34) grip on the head (16) of the bone screw (12), wherein the two jaws (30, 34) are connected together pivotably and not crossing each other via a pivot axis (40) lying between the distal and proximal ends (42, 44, 46, 48) and running orthogonally to the longitudinal axis (38) of the housing (22), so that pressing together the proximal ends (42, 44) of the jaws (30, 34) causes an opening of the distal ends (46, 48) of the jaws (30, 34), **characterised in that** the internal thread (52) of the housing (22) is formed at the proximal end (42) of a single jaw (30), **in that** the proximal end (42) of this jaw (30) protrudes over the proximal end (44) of the other jaw (34) in the direction of the longitudinal axis (38) by a portion (32) carrying the internal thread (52).

2. Instrument according to any of the preceding claims, **characterised in that** one jaw (30) is rigid and one jaw (34) is pivotable relative to the longitudinal axis (38) of the internal thread (52).

3. Instrument according to any of the preceding claims, **characterised in that** the one jaw (30) on which the internal thread (52) of the housing (22) is formed is configured as a single monolithic piece, and together with the other jaw (34) forms the housing (22).

4. Instrument according to any of the preceding claims, **characterised in that** the adjustment tool (24) is structured in at least two pieces in the direction of the longitudinal axis (38) and has a rotatable portion (58) provided with the external thread (54) and a portion (60) acting on the spinal column rod and held rotationally fixedly on the housing (22).

5. Instrument according to claim 5, **characterised in that** the portion (60) held rotationally fixedly on the housing (22) is guided in the direction of the longitudinal axis (38) and between the two jaws (30, 34).

6. Instrument according to any of the preceding claims, **characterised in that** when the proximal ends (42, 44) of the jaws (30, 34) are pressed together, the jaw (34) engages in the thread alignment of the external thread (54) of the adjustment tool (24).

7. Instrument according to any of the preceding claims, **characterised in that** the external thread (54) of the adjustment tool (24) has a diameter which is greater than the spacing between the pressed-together proximal ends (42, 44) of the jaws (30, 34).

8. Instrument according to any of the preceding claims, **characterised in that** the housing (22) and/or the adjustment tool (24) has receivers (92, 100) for additional holding (94) and/or turning handles (102).

## Revendications

1. Instrument (10) pour la liaison d'une vis à os (12) avec une broche spinale (20), l'instrument (10) comprenant un boîtier (22) globalement cylindrique et creux ainsi qu'un outil de réglage (24) traversant axialement le boîtier (22), qui comprend, au niveau d'une portion (56), un filetage externe (54) et est vissé dans un filetage interne (52) du boîtier (22), le filetage interne (52) définissant un axe longitudinal (38) du boîtier (22) et le boîtier (22) étant conçu sous la forme d'une pince et comprenant une première et une deuxième mâchoire (30, 34), qui constituent le boîtier (22) et qui comprennent chacune, par rapport à l'utilisateur, une extrémité proximale et une extrémité distale (42, 44, 46, 48), les extrémités proximales (42, 44) des mâchoires servant à la saisie de l'instrument par l'utilisateur et les extrémités distales (46, 48) des mâchoires (30, 34) s'emboîtant avec la tête (16) de la vis à os (12), les deux mâchoires (30, 34) étant reliées de manière articulée et entre elles sans se croiser par l'intermédiaire d'un axe de pivotement (40) s'étendant entre les extrémités distales et proximales (42, 44, 46, 48) et orthogonales à l'axe longitudinal (28) du boîtier (22), de façon à ce qu'une compression des extrémités proximales (42, 44) des mâchoires (30, 34) provoque une ouverture des extrémités distales (46, 48) des mâchoires (30, 34), **caractérisé en ce que** le filetage interne (52) du boîtier (22) est réalisé sur l'extrémité proximale (42) d'une seule mâchoire (30), grâce au fait que l'extrémité proximale (42) de cette mâchoire (30) dépasse de l'extrémité proximale (44) de l'autre mâchoire (34) en direction de l'axe longitudinal (38) avec une portion (32) supportant le filetage interne (52).

2. Instrument selon l'une des revendications précédentes, **caractérisé en ce qu'**une mâchoire (30) est rigide et une mâchoire (34) est pivotante par rapport à l'axe longitudinal (38) du filetage interne (52).

3. Instrument selon l'une des revendications précédentes, **caractérisé en ce que** la mâchoire (30), sur laquelle est réalisée le filetage interne (52) du boîtier (22), est conçue comme une partie constituée d'une seule pièce et constitue, avec l'autre mâchoire (34), le boîtier (22).

4. Instrument selon l'une des revendications précédentes, **caractérisé en ce que** l'outil de réglage (24) est conçu, en direction de l'axe longitudinal (38), au moins en deux parties et comprend une portion (58) rotative munie du filetage externe (54) et une portion (60) s'emboîtant sur la broche spinale et maintenue de manière bloquée en rotation sur le boîtier (22).

5. Instrument selon la revendication 5, **caractérisé en ce que** la portion (60), maintenue de manière bloquée en rotation sur le boîtier (22), est guidée en direction de l'axe longitudinal (38) et ente les deux mâchoires (30, 34).

6. Instrument selon l'une des revendications précédentes, **caractérisé en ce que** lors de la compression des extrémités proximales (42, 44) des mâchoires (30, 34), la mâchoire (34) s'emboîte dans le filet du filetage externe (54) de l'outil de réglage (24).

7. Instrument selon l'une des revendications précédentes, **caractérisé en ce que** le filetage externe (54) de l'outil de réglage (24) présente un diamètre qui est supérieur à la distance entre les extrémités proximales (42, 44) comprimées des mâchoires (30, 34).

8. Instrument selon l'une des revendications précédentes, **caractérisé en ce que** le boîtier (22) et/ou l'outil de réglage (24) présente des logements (92, 100) pour des poignées de maintien (94) et/ou des poignées rotatives (102) supplémentaires.
